# EUROPÄISCHE PATENTSCHRIFT

(11) **EP 1 154 784 B1**
(45) Veröffentlichungstag und Bekanntmachung des Hinweises auf die Patenterteilung: **23.10.2002**
(21) Anmeldenummer: 00912494.2
(22) Anmeldetag: 22.02.2000
(51) Int. Cl.: A61K 38/06, A61K 31/52, A61P 31/22

(54) **THERAPEUTISCH WIRKSAMES STOFFGEMISCH ENTHALTEND S-ACETYLGLUTATHION UND ACICLOVIR**
THERAPEUTICALLY ACTIVE MIXTURE OF SUBSTANCES CONTAINING S-ACETYLGLUTATHIONE AND ACICLOVIR
MELANGE DE SUBSTANCES A ACTION THERAPEUTIQUE CONTENANT DU S-ACETYLGLUTATHION ET DE L'ACICLOVIR

(30) Priorität: 22.02.1999 DE 19907507
(43) Veröffentlichungstag der Anmeldung: 21.11.2001
(73) Patentinhaber: Treusch, Gernot, Castello d'Empuries (ES)
(72) Erfinder: Treusch, Gernot, Castello d'Empuries (ES)
(74) Vertreter: Lieck, Hans-Peter, Dipl.-Ing.
(86) Internationale Anmeldenummer: EP0001419
(87) Internationale Veröffentlichungsnummer: WO00050060

(56) Entgegenhaltungen:
- WO-A-92/03146
- GB-A- 761 093
- O'BRIEN W J ET AL: "Effects of nucleoside antivirals and their metabolites on the corneal endothelium." CURRENT EYE RESEARCH, (1981) 1 (4) 243-8. , XP000925158
- KUROKAWA M ET AL: "EFFICACY OF TRADITIONAL HERBAL MEDICINES IN COMBINATION WITH ACYCLOVIR AGAINST HERPES SIMPLEX VIRUS TYPE 1 INFECTION IN VITRO AND IN VIVO" ANTIVIRAL RESEARCH,NL,ELSEVIER SCIENCE BV., AMSTERDAM, Bd. 27, Nr. 1/02, 1995, Seiten 19-37, XP000571856 ISSN: 0166-3542
- PANCHEVA S. ET AL: "Effect of combined acyclovir and ribavirin on experimental Herpes simplex virus type 1 keratoconjunctivitis in rabbits" ACTA MICROBIOLOGICA BULGARICA, Bd. 29, 1993, Seiten 61-64, XP000911006
- MUCSI I ET AL: "COMBINED EFFECTS OF FLAVONOIDS AND ACYCLOVIR AGAINST HERPESVIRUSES IN CELL CULTURES" ACTA MICROBIOLOGICA HUNGARICA,HU,AKAD. KIADO, BUDAPEST, Bd. 39, Nr. 2, 1992, Seiten 137-147, XP002074629 ISSN: 0231-4622
- HOLLIDAY J ET AL: "Inhibition of herpes simplex virus types 1 and 2 replication in vitro by mercurithio analogs of deoxyuridine." ANTIVIRAL RESEARCH, (1991 SEP) 16 (2) 197-203. , XP000925125
- FIELD H J ET AL: "EFFECT OF ACYCLOGUANOSINE TREATMENT ON ACUTE AND LATENT HERPES SIMPLEX INFECTIONS IN MICE" ANTIMICROBIAL AGENTS AND CHEMOTHERAPY,US,AMERICAN SOCIETY FOR MICROBIOLOGY, WASHINGTON, DC, Bd. 15, Nr. 4, 1. April 1979 (1979-04-01), Seiten 554-561, XP000568622 ISSN: 0066-4804

## Beschreibung

Aus der EP 0 327 612 B1 ist die Verwendung von S-Acetylglutathion als Arzneimittel bei verschiedenen Indikationen bekannt. S-Acetylglutathion ist der Mono-Acetyl-(thio)-ester des Glutathions (gamma-Glutamyl-Cysteinyl-Glycin) mit folgender Strukturformel:

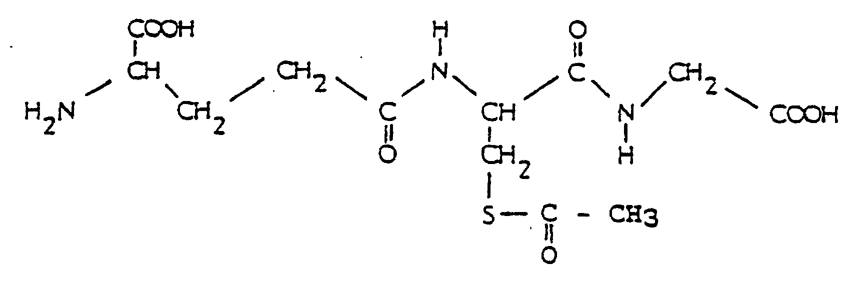

Ein Verfahren zur Herstellung von S-Acetylglutathion ist in der US 2,760,956 offenbart.

Aciclovir (ACV) ist ein bekanntes Arzneimittel gegen den Herpes Simplex Virus (HSV). Aciclovir steht als Kurzbezeichnung (INN) für 9-[(2-Hydroxyethoxy)methyl]guanin.

Es wurde nun erkannt, daß überraschenderweise die Kombination von S-Acytylglutathion und ACV einen starken synergistischen Effekt gegen den Herpes Simplex Virus (HSV), insbesondere gegen HSV-1 hat, außerdem gegen den Varizella Zoster Virus (VZV).

Durchgeführt wurden in vitro Untersuchungen zum Einfluß von S-Acetylglytathion und von Aciclovir (ACV), jeweils einzeln und in Kombination miteinander, auf die HSV-1 Replikation in humanen Vorhaut-Fibroblasten mit MEM plus 10% fötales Kälberserum als Medium. Dabei wurden zwei verschiedene Konzentrationen des S-Acetylglutathion und drei verschiedene Konzentrationen des ACV verwendet. Die Ergebnisse sind in Tabelle 1 dargestellt:

S-Acetylglutathion ist ab einer Konzentration von 10 mM (Millimol/Liter) gegenüber HSV-1 wirksam. ACV allein zeigt erwartungsgemäß eine deutliche Wirksamkeit, insbesondere in der Konzentration von 2 µM (Micromol/Liter). Die Kombination von S-Acetylglutathion und ACV ergibt einen starken synergistischen Anti-HSV-1 Effekt. Wird S-Acetylglutathion in einer Konzentration von 20 mM zusammen mit ACV in einer Konzentration von 2 µM eingesetzt, ist ein Virustiter nicht mehr nachweisbar.

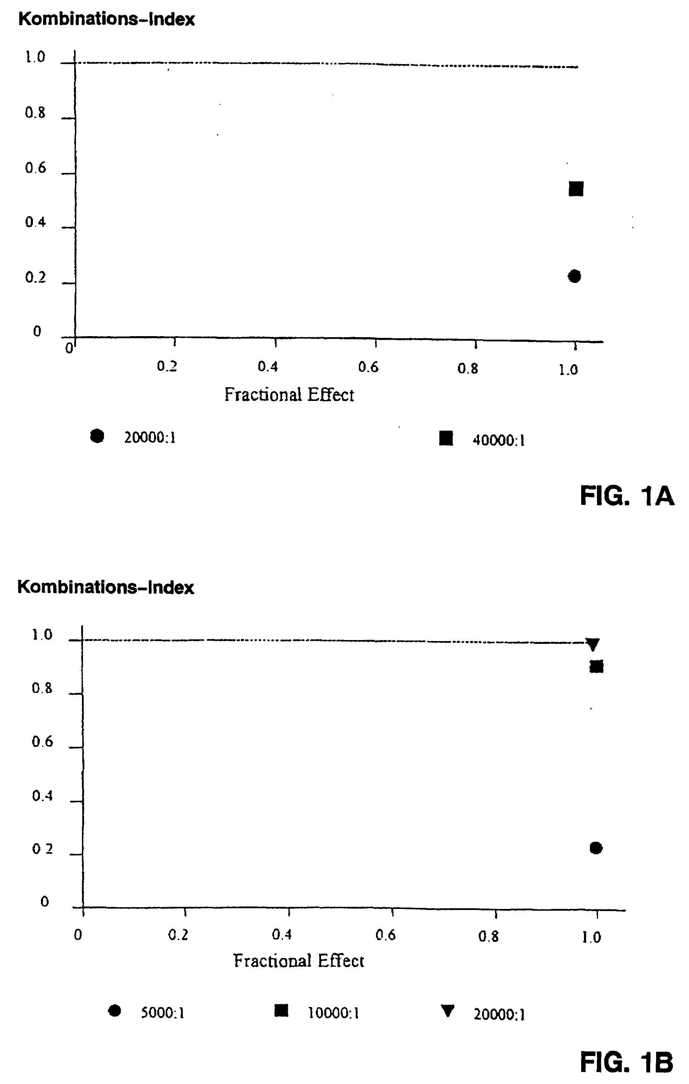

Der synergistische Effekt einer Kombination von S-Acetylglutathion und ACV gemäß Tabelle 1 ist in den Figuren 1a und 1b nach einem bekannten mathematischen Modell grafisch dargestellt.

Figur 1a bezieht sich auf die Konzentration des S-Acetylglutathions von 20 mM, Figur 1b auf die Konzentration von 10 mM. Für die verschiedenen Konzentrations-Verhältnisse von S-Acetylglutathion zu ACV, mit Ausnahme des eindeutigen Falles 20 mM/2 µM, sind Punkte in einem Koordinatensystem aufgetragen, dessen Y-Achse den Kombinations-Index wiedergibt. Bei dem verwendeten Auswertungsmodell bedeutet ein Kombinations-Index von kleiner als 1 einen synergistischen Effekt, ein Kombinations-Index gleich 1 einen additiven Effekt und ein Kombinations-Index von größer als 1 einen antagonistischen Effekt. Man sieht, daß alle verwendeten Kombinationen im synergistischen Bereich liegen.

Es wurden ferner durchgeführt in vitro Untersuchungen zum Einfluß von S-Acetylglutathion und Aciclovir (ACV), jeweils einzeln und in Kombination miteinander, auf die VCV (Strain 4400/95) Replikation in Retinal pigmentierten Epitelzellen (RPE) mit IMDM plus 10% fötales Kälberserum als Medium. Die

### Ergebnisse sind in Tabelle 2 wiedergegeben:

Bei einer Konzentration von 10 mM zeigt S-Acetylglutathion zusammen mit ACV in drei verschiedenen Kozentrationen einen ausgeprägten synergistischen Effekt, der bei einer Konzentration des ACV von 40 mM am höchsten ist. Bei dieser Kombination sinkt die Anzahl der Plaques oder Löcher, die unter dem Einfluß des VCV entstehen, unter die Nachweisbarkeits-Grenze.

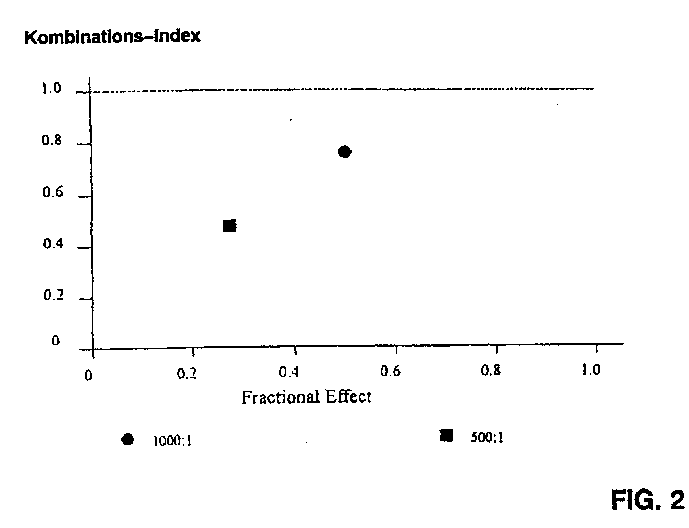

Figur 2 zeigt den synergistischen Effekt nach dem gleichen Modell, das für die Figuren 1a und 1b verwendet wurde.

Ein Pharmazeutikum gegen den Herpes Simplex Virus enthält die beiden Substanzen S-Acetylglutathion und ACV vorzugsweise in einer Creme- oder Salben-Grundlage zur äußeren Anwendung und in den Konzentrationen, die sich nach den obigen Versuchen als besonders wirksam erwiesen haben.

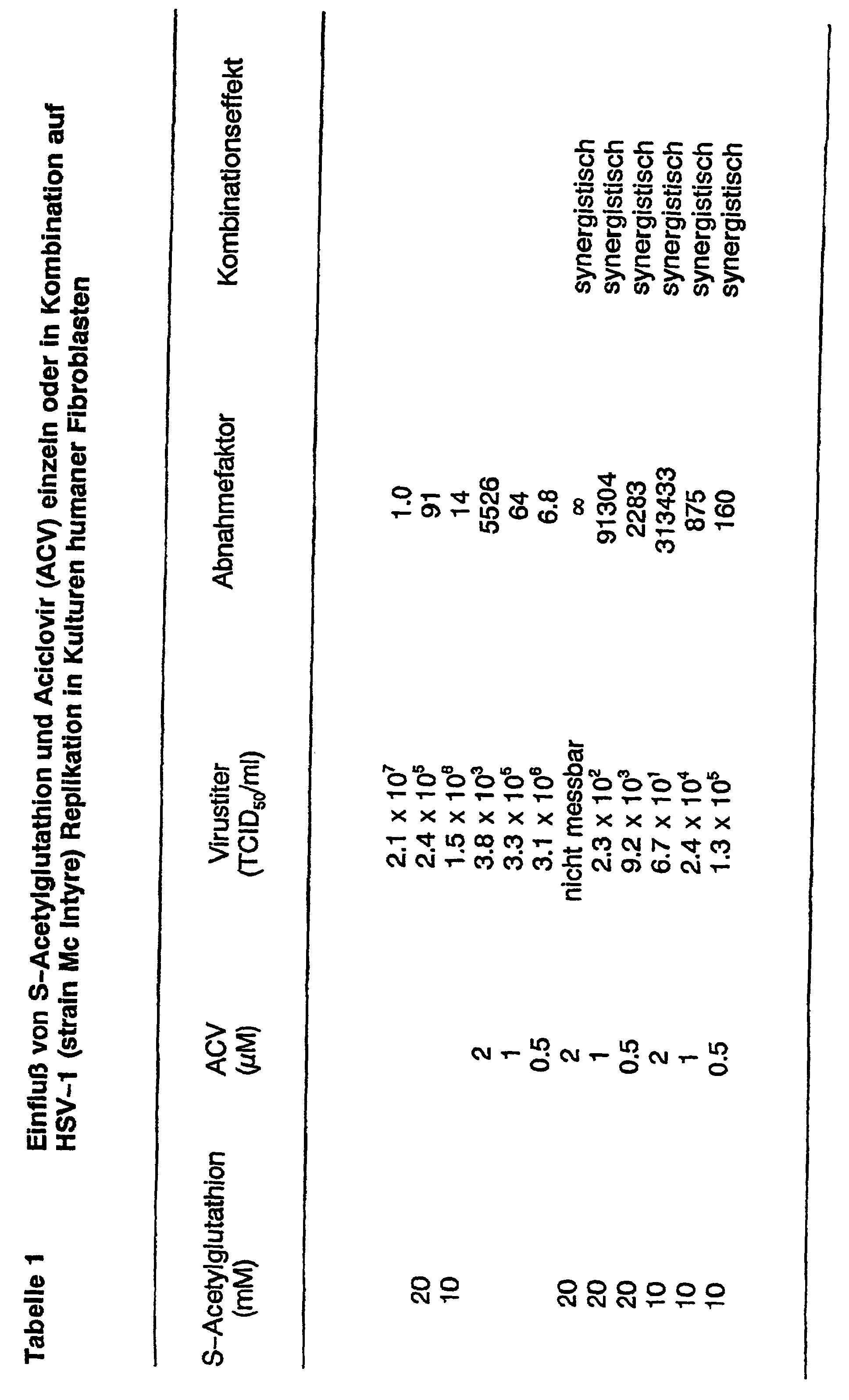

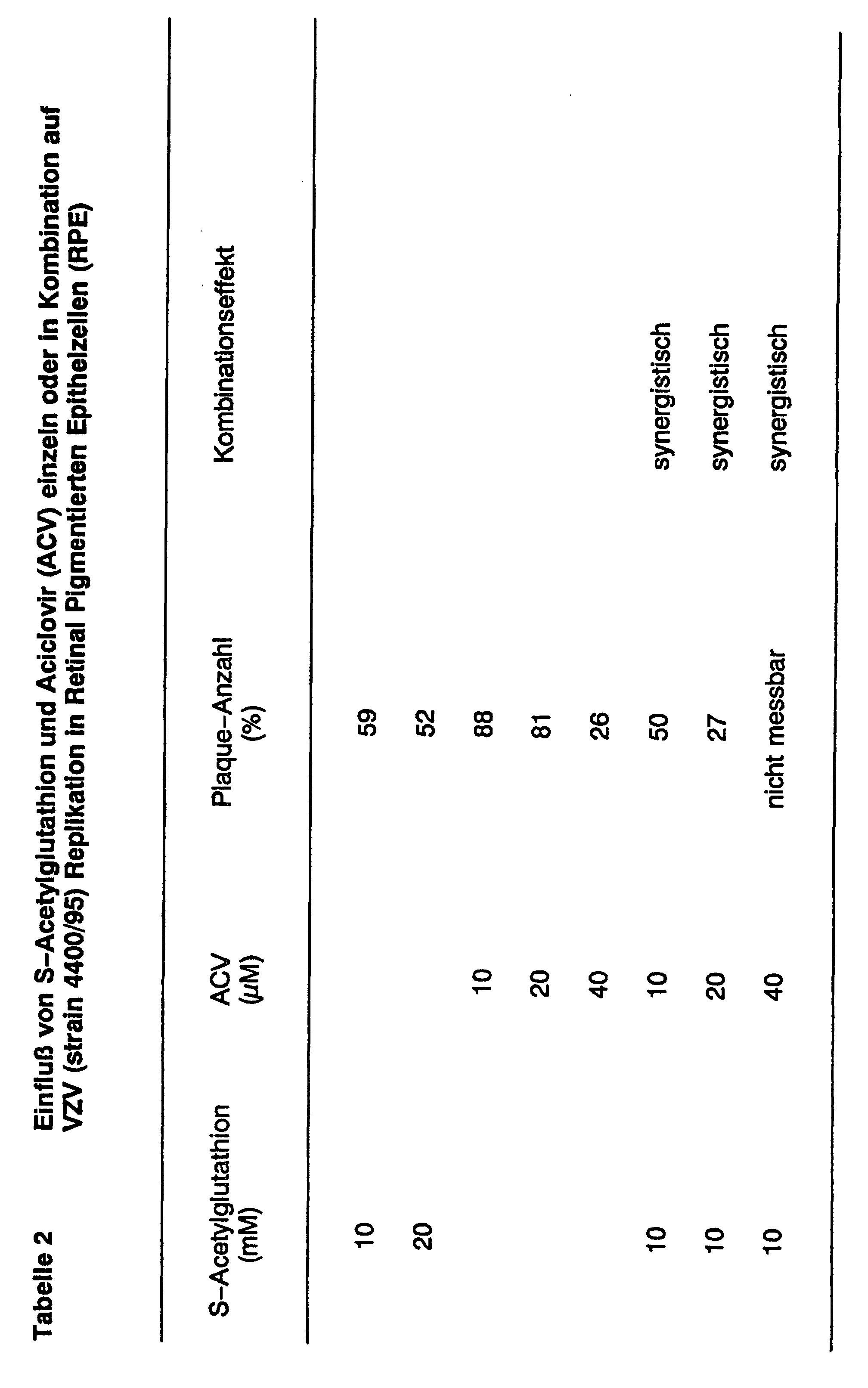

## Patentansprüche

1. Stoffgemisch enthaltend S-Acetylglutathion und Aciclovir als Arzneimittel gegen den Herpes Simplex-Virus oder den Varizella Zoster Virus.

2. Verwendung von S-Acetylglutathion in Kombination mit Aciclovir zur Herstellung eines Arzneimittels, das gegen Infektionen mit dem Herpes Simplex-Virus oder dem Varizella Zoster Virus wirksam ist.

## Claims

1. A substance mixture containing S-acetyl glutathione and Aciclovir as medication against the Herpes Simplex virus or the Varicella Zoster virus.

2. A use of S-acetyl glutathione in combination with Aciclovir for preparing a medication which is effective against infection with the Herpes Simplex virus or the Varicella Zoster virus.

## Revendications

1. Mélange contenant du glutathion d'acétylène S et de l'aciclovir comme médicament contre le virus simplex de l'herpès ou le virus zoster de la varicelle.

2. Utilisation de glutathion d'acétylène S en combinaison avec de l'aciclovir pour la fabrication d'un médicament qui est actif contre les infections au virus simplex de l'herpès ou au virus zoster de la varicelle.
